# EUROPEAN PATENT SPECIFICATION

(11) **EP 0 421 984 B1**
(45) Date of publication and mention of the grant of the patent: **05.07.1995**
(21) Application number: 88904051.5
(22) Date of filing: 30.03.1988
(51) Int. Cl.: B25G 1/10, A61M 35/00, A45D 40/26

(54) **APPLICATOR AND PACKAGE THEREFOR**
APPLIKATOR UND DESSEN VERPACKUNG
APPLICATEUR ET SON EMBALLAGE

(43) Date of publication of application: 17.04.1991
(73) Proprietor: SHABO, Alan L., Los Angeles, CA 90024 (US)
(72) Inventor: SHABO, Alan L., Los Angeles, CA 90024 (US)
(74) Representative: Allman, Peter John
(86) International application number: US8801093
(87) International publication number: WO8909118

(56) References cited:
- EP-A- 0 244 156
- GB-A- 1 055 471
- US-A- 982 232
- US-A- 3 126 006
- US-A- 3 168 072
- US-A- 3 324 849
- US-A- 4 037 975
- US-A- 4 213 472
- US-A- 4 517 701

## Description

The present invention relates to an applicator for scrubbing and for applying liquid to an eyelid, as defined in the pre-characterising portion of Claim 1.

The pre-characterising portion of Claim 1 is based on the disclosure of the document EP-A-244156.

A common, chronic or recurrent condition of the eye is inflammation of the eyelid margins, known medically as blepharitis. The condition is characterized by tenacious deposits on and around the eyelashes of an oily secretion (mild, simply blepharitis) and crusty desquamations or scales (seborrheic or squamous blepharitis). Blockage of the oil gland ducts at the eyelid margin may result in secondary infection with swollen, red-rimmed or ulcerated eyelids and styes (staphylococcal blepharitis). Blepharitis is particularly troublesome to contact lens wearers when their contact lenses become coated and must be removed frequently for cleaning or replaced. In individuals with bacterial or viral conjunctivitis, there is often a pus or mucous discharge that causes the eyelids to stick together. Furthermore, in patients following eye operations, the eyelashes often become coated with medications or excessive discharge.

An important procedure in relieving such problems is cleansing of the eyelids to remove the deposits (oily secretion, scales, pus, mucus and eye medications). Indeed, daily cleansing with a mold dilute soap solution is found to completely alleviate the milder forms of blepharitis. However, attempts to apply soap and scrub the eyelid using a cotton swab or a swab stick, such as is sold under the trademark Q-tip, can lead to some undesirable side effects or consequences. This is particularly true when attempting to remove extensive or tenacious discharge from the eyelid margins.

One undesirable and potentially dangerous side effect of using a cotton swab or swab stick is due to the fact that the thin stick of a cotton swab stick is difficult to hold firmly, particularly in close proximity to the eye-ball. Due to its narrow, rounded shape, it tends to roll between the forefinger and thumb. Accordingly, the patient will often grasp the stick at its free end, with the consequent danger of poking the eye and scratching the cornea. Furthermore, the narrow, pointed top of a cotton swab stick is difficult to apply directly to the eyelid margin. Patients often use the side of the cotton swab which causes the narrower tip to override the eyelid margin, further increasing the danger of scratching the cornea. Another undesirable side effect occurs when the patient uses the same swab or swab stick to remove discharge from both eyes. Since blepharitis is often associated with conjunctivitis, such practice can lead to transferring an infection from one eye to the other. In addition, a high concentration of soap can accidentally be applied to the eyelid, enter the tear fluid, and cause burning, stinging and possible damage to the sensitive corneal cells that line the eyeball and assist in vision. Finally, in scrubbing with cotton swabs not specifically made for application around the eye, filaments of cotton can loosen and become affixed to the eye-lashes or actually shed into the inside of the eyelid causing irritation, infection and/or damage to the eyeball.

US-A-4213472 discloses an applicator which fits over a user's finger. Thus it does not have a handle which is intended to be grasped between a user's thumb and forefinger, and therefore cannot enable an accurate and yet gentle application of a swab to the delicate tissues of the eyelid.

US-A-3324849 discloses a combination tongue depressor and swab. The device has a long handle appropriate to its intended use, but such a handle is not suitable for positioning and moving a swab in proximity to the eye.

EP-A-244156 discloses a swab applicator with a thin, long handle which cannot be readily grasped between a user's thumb and forefinger in a manner which would permit accurate positioning and movement of the swab in proximity to the eye.

GB-A-1055471 discloses a device for applying a swab to the nasal and sinus canals. It is in the form of a flattened but narrow long strip of material to one end of which a swab is attached. Such a device could not be easily gripped or manoeuvred by a user grasping it between thumb and forefinger in a manner which would permit accurate positioning and movement of the swab in proximity to the eye.

The foregoing problems are overcome by the present invention which provides an applicator designed specifically to scrub and to apply liquid to an eyelid, and a sealed, contaminant-excluding package for the applicator. The applicator is formed so as to provide controlled, safe movement near the eyeball along the eyelid margin and to facilitate the application of liquid to the eyelid as well as the removal of tenacious deposits. In this regard, it comprises a generally flat handle formed to be grasped between the thumb and forefinger of the user. The handle has front and rear major surfaces joined by a minor, edge surface, the length and width of the handle being each substantially greater than its depth. An elongate peg extends outwardly from an edge of the handle and a swab is disposed on the peg adjacent the handle edge. The swab is formed of liquid absorbent material, and has a broad flat tip for optimal application to the eyelid margin. The swab is disposed so that the distance between the swab and the handle edge is substantially less than either the width or length of the handle. Preferably, the swab has greater width than depth with the handle, peg and swab on a common longitudinal axis so that the swab lies substantially in the plane of the handle. The result is an applicator which can be easily grasped between the thumb and forefinger for optimal control of movement near the eyeball. When applied to the eyelid, the broad flat swab tip naturally angles properly close to the lash line to dispose liquid solution, such as a mild soap solution, and to remove tenacious deposits from the eyelid margin. Ridges or depressions can be formed so as to be exposed on major surfaces of the handle to further facilitate grasping between the thumb and forefinger. This, together with wrist movement to and fro, allows controlled movement back and forth along the eyelid margin without the danger of forward thrust toward the eyeball.

Preferably, the applicator is packaged with the desired liquid already absorbed in safe and proper concentration for use near the eyeball in the swab. By pre-packaging the liquid with the swab, a single dose application can be provided for, so that the patient does not use the same swab on both eyes. It thus encourages the patient to use a separate swab for each eye, avoiding the transfer of conjunctivitis or other infection from one eye to the other. An antibacterial or antibiotic agent can be incorporated in the liquid.

In packaging the applicator, a sealed, contaminant-excluding packaging unit is provided. A blister pack can be used in which a flexible polymeric material is releasably sealed to a peel-away backing. The polymeric material of such a blister pack closely conforms to the shape of the applicator whereby to substantially isolate the handle from the swab. If any liquid does penetrate into the handle compartment, the flatness and thinness of the handle minimizes the adherence of liquid. In another embodiment, a plurality of swabs containing the liquid absorbed therein are packaged along with a single handle, each separated from the other. Means are provided for removably connecting a selected swab to the handle as a disposable swab. After the swab is used, it is removed from the handle, and thereafter the handle is connected to another swab for use in the other eye or at another time. In this regard, the handle can be formed integrally with a peg and the swabs can be each formed with an opening for receiving the peg. Alternatively, each of the swabs can be disposed on the end of its own peg, which can be press-fit into an opening formed in the handle.

It will be appreciated that the inventive concepts herein have application not only to the medical treatment of blepharitis or other such conditions, but also to simple cosmetic use. For example, because the applicator is designed to be easily manipulated with respect to the eyelid, it can be used to remove eye makeup with minimum danger to the eye. The applicator can be provided as such without any liquid associated therewith, or it can be packaged with a liquid that facilitates removal of eye makeup. The construction of the applicator is quite economical, encouraging single dose usage, particularly when it is pre-packaged with the eye makeup removal liquid, therefore discouraging the use of the same swab on both eyes, and also discouraging the sharing of the swab among family members.

### BRIEF DESCRIPTION OF THE DRAWINGS

Figure 1 illustrates use of an applicator of the present invention to scrub and to apply liquid on a patient's eyelid;
Figure 2 is a plan view of an applicator formed in accordance with the first embodiment of the present invention;
Figure 2A is an alternate embodiment of the applicator;
Figure 3 is an elevational view of the applicator, taken on line 3-3 of Figure 2;
Figure 4 is an elevational view, partially in cross-section, of the applicator tip, taken approximately on line 4-4 of Figure 2;
Figure 5 is a plan view of a blister pack package of ten applicators;
Figure 6 is a cross-sectional view of one of the package units of Figure 5, taken along line 6-6 of Figure 5;
Figure 7 is a plan, exploded view of an applicator formed in accordance with the second embodiment of the invention;
Figure 8 is a package of the applicator handle of Figure 7 and six swabs of Figure 7;
Figure 9 is a plan, exploded view of an applicator formed in accordance with another embodiment of the invention; and
Figure 10 is an elevated view of the front of the applicator handle of Figure 9, taken on line 10-10 of Figure 9.

### DETAILED DESCRIPTION

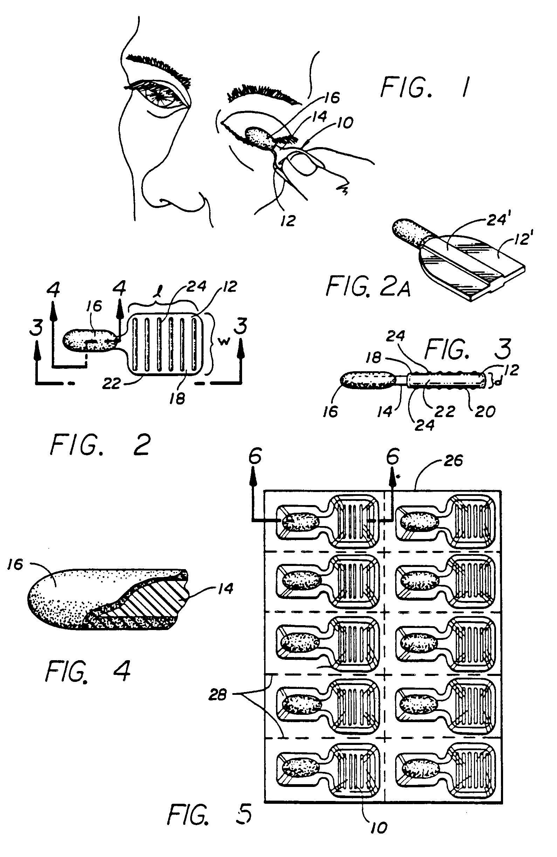

Referring to Figure 1, use of the applicator 10 is shown. The applicator 10 includes a handle formed with an elongate peg 14 on which is disposed a swab 16 in flattened form with rounded corners. The applicator is applied so that the swab lies flat on the eyelid, a position which is facilitated by the arrangement of the components as will be described in more detail hereinafter.

A first embodiment of the applicator is illustrated in Figures 1 through 6. Referring to Figures 2 and 3, the handle 12 of the applicator is formed with front and rear major surfaces 18 and 20 joined by a minor, edge surface 22. As indicated by the letters "l" and "w" in Figure 2 and "d" in Figure 3, the length and width of the handle 12 are each substantially greater than its depth. A plurality of ridges 24 are formed on the major surfaces of the handle, either on one side, or on both sides as shown, to facilitate the ability to grasp the handle between the thumb and forefinger. In place of ridges 24, one can use grooves.

Figure 2A illustrates an alternate embodiment wherein the generally flat handle 12' is formed with a raised central region 24' on each side (only one side being shown in the drawing).

As shown in shadow in Figure 3, the peg 14 is elongate and extends outwardly from the edge 22 of the handle 12. Referring additionally to Figure 4, the swab 16 is disposed on the peg 14 so as to lie adjacent to the edge 22 of the handle 12. The swab can be formed of any liquid absorbent material, for example, a porous sponge material, or a cotton backing.

As shown in Figures 2 and 3, the swab 16 has greater width than depth, and is disposed on a common longitudinal axis so that the plane of the swab lies substantially in the plane of the handle. Also, in the embodiment of Figures 1-6, as shown, the length dimension of the handle is less than twice the width dimension thereof. This facilitates use of the applicator to scrub and apply liquid near the eye as one unit for optimal control of movement.

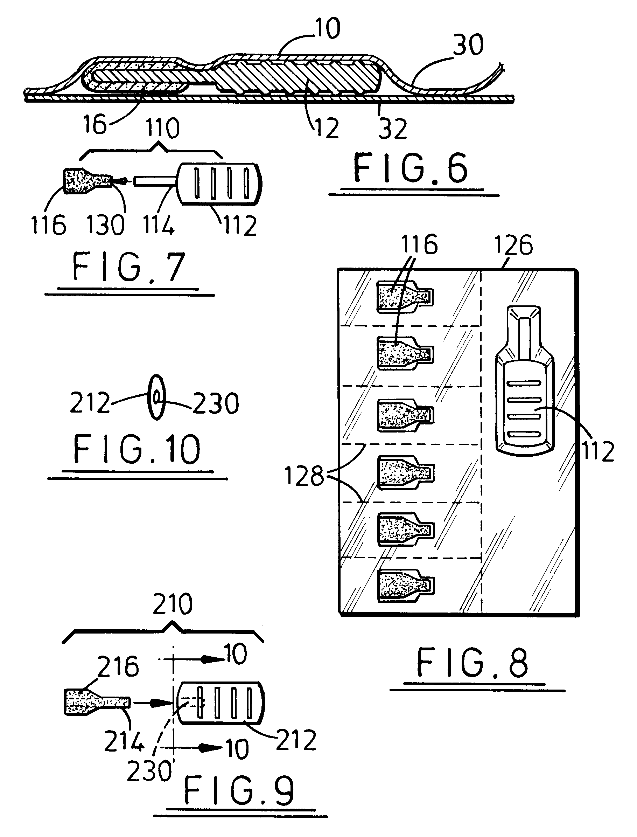

Referring to Figure 5, a blister-pack package 26 of applicators 10, is shown. This particular package accommodates ten applicators 10, with liquid absorbed in the swab 16, each in its individual sealed, contaminant-excluding packaging unit defined by tear lines of weakness 28. Referring to Figure 6, an individual unit is shown in cross-section in which a blister pack, flexible polymeric material 30 is releasably sealed to a peel-away paper backing 32 so as to encase an applicator 10. The polymeric material closely conforms to the shape of the applicator so as to substantially isolate the handle 12 from the swab 16. As shown in Figure 6, this isolation is facilitated by coaction of at least one ridge with the closely conformed polymeric material. Because of the unique shape of the handle 12, even if liquid were to run off from the swab 16 and migrate into the handle 12 compartment, only minimum wetting of the handle would occur. The generally flat surface together with the ridges or grooves on the major surfaces would facilitate grasping even when the handle was wet.

Referring to Figure 7, there is shown an applicator 110 constructed in accordance with another embodiment. The applicator 110 includes a handle 112. It is formed at one edge with an integral peg 114 which is designed to fit into a disposable swab 116. The swab 116 is formed of a sponge-like material having an opening 130 at one end to receive the peg 114.

The peg 114 can be oval-shaped to match an oval-shaped opening in the swab 116 so as to ensure that the swab 116 is oriented in a plane substantially in the plane of the handle 112.

Referring to Figure 8, a sealed, contaminant-excluding package is shown in which a plurality of swabs 116 are contained in a single blister pack unit. The swab tips include a liquid, such as a mild soap solution, absorbed therein. While a plurality of swabs 116 are provided, a single handle 112 is provided encased in its own blister pack compartment. The result is a package 126 in which the individual swabs are sequentially removable by means of tear lines of weakness 128. This package has the advantage of tremendous economy as the swabs are disposable following single eyelid application while the handle is reusable.

Referring to Figures 9 and 10, there is shown an applicator 210 formed in accordance with still another embodiment of the invention. In this embodiment, the handle 212 is not formed integrally with a peg. Rather, a peg 214 is connected directly to each swab 216 so as to be an integral part thereof. This construction is particularly useful when it is desired to use cotton batting closely wound around the peg 214. In this case, the handle 212 is formed with an opening 230, shown in shadow in Figure 9, into which the peg 214 can be press-fit.

The peg 114 or 214 can be oval-shaped to match an oval-shaped opening in the swab 216 so as to ensure that the swab is oriented in a plane substantially in the plane of the handle 212.

In common with all of the illustrated embodiments, as shown in the drawings, the handle is thicker on at least a portion of the longitudinal axis than along the handle edge. Such thickness is provided by one or more ridges 24 as in Figures 2, 7 and 9, or by the raised central portion 24' in Figure 2A. Such structure facilitates the above-described controlled, safe movement of the applicator near the eyeball along the eyelid margin so that a scrubbing action can be safely accomplished.

## Claims

1. An applicator (10, 110, 210) for scrubbing, and for applying liquid to an eyelid, comprising a thin one-piece handle (12, 112, 212) formed to be grasped between the thumb and forefinger of a user, a swab (16, 116, 216) formed of liquid absorbent material mounted on said handle, said swab and said handle each having a length dimension defining an axis of the applicator, a width dimension in a first plane and a thickness dimension transverse of said first plane, the length of the handle being substantially greater than its width and thickness, and the length of the swab being greater than its width and thickness, characterised in that the handle is flat-sided to define an edge, said swab is mounted on a peg (14, 114, 214) which extends outwardly entirely perpendicularly from said edge of said handle along the applicator axis, the swab has a width greater than its thickness, the distance between said swab and said handle edge is substantially less than either the width or length of said handle, the width of said handle is greater than the width of said swab, the width of said handle is substantially greater than its thickness, and the length of said handle is less than twice its width, thereby to permit a user to freely grasp said handle between the users thumb and forefinger adjacent said handle edge so that the users thumb can point generally in the direction of the eyelid.

2. An applicator according to claim 1, including a plurality of ridges (24) exposed on the flat sides of said handle.

3. An applicator according to claim 1 or 2, including a liquid absorbed in said swab.

4. An applicator according to claim 1, 2 or 3, in which said handle is thicker on at least a medial portion thereof than along an edge thereof.

## Patentansprüche

1. Applikator (10, 110, 210) zum Reinigen durch Reiben und zum Auftragen von Flüssigkeit auf ein Augenlid, der folgendes umfaßt: einen dünnen einteiligen Griff (12, 112, 212), der geformt ist, um zwischen Daumen und Zeigefinger eines Benutzers gehalten zu werden, einen aus flüssigkeitsabsorptivem Material geformten, am genannten Griff befestigten Tupfer (16, 116, 216), wobei der genannte Tupfer und der genannte Griff jeweils ein eine Achse des Applikators definierendes Längenmaß, ein Breitenmaß in einer ersten Ebene und ein Dickenmaß quer zur genannten ersten Ebene haben, wobei die Länge des Griffs wesentlich größer ist als seine Breite und Dicke und die Länge des Tupfers größer ist als seine Breite und Dicke, dadurch gekennzeichnet, daß der Griff flachseitig ist, um eine Kante zu definieren, der genannte Tupfer auf einem Zapfen (14, 114, 214) befestigt ist, der nach außen völlig senkrecht von der genannten Kante des genannten Griffs entlang der Applikatorachse verläuft, der Tupfer eine Breite hat, die größer ist als seine Dicke, der Abstand zwischen dem genannten Tupfer und der genannten Griffkante wesentlich geringer ist als die Breite oder die Länge des genannten Griffs, die Breite des genannten Griffs größer ist als die Breite des genannten Tupfers, die Breite des genannten Griffs wesentlich größer ist als seine Dicke und der genannte Griff weniger als zweimal so lang ist wie breit, wodurch ein Benutzer den genannten Griff neben der genannten Griffkante frei zwischen seinem Daumen und seinem Zeigefinger halten kann, so daß der Daumen des Benutzers allgemein in Richtung des Augenlids weisen kann.

2. Applikator nach Anspruch 1, der eine Mehrzahl von auf den flachen Seiten des genannten Griffs freiliegenden Rippen (24) aufweist.

3. Applikator nach Anspruch 1 oder 2, der eine im genannten Tupfer absorbierte Flüssigkeit aufweist.

4. Applikator nach Anspruch 1, 2 oder 3, bei dem der genannte Griff zumindest an einem nach der Mitte zu gelegenen Abschnitt davon dicker ist als entlang einer Kante davon.

## Revendications

1. Applicateur (10, 110, 210) servant à frotter, et à appliquer du liquide sur une paupière, comprenant un bâtonnet mince en une pièce (12, 112, 212) formé pour être saisi entre le pouce et l'index d'un utilisateur, un tampon (16, 116, 216) constitué par une matière absorbante de liquide monté sur ledit bâtonnet, ledit tampon et ledit bâtonnet ayant chacun une dimension en longueur définissant un axe de l'applicateur, une dimension en largeur dans un premier plan et une dimension en épaisseur transversale par rapport audit premier plan, la longueur du bâtonnet étant sensiblement supérieure à sa largeur et à son épaisseur, et la longueur du tampon étant supérieure à sa largeur et son épaisseur, caractérisé en ce que le bâtonnet présente un côté aplati pour définir une arête, ledit tampon est monté sur une cheville (14, 114, 214) qui s'étend vers l'extérieur complètement perpendiculairement depuis ladite arête dudit bâtonnet le long de l'axe de l'applicateur, le tampon a une largeur supérieure à son épaisseur, la distance entre ledit tampon et ladite arête du bâtonnet est sensiblement inférieure à la largeur et à la longueur dudit bâtonnet, la largeur dudit bâtonnet est supérieure à la largeur dudit tampon, la largeur dudit bâtonnet est sensiblement supérieure à son épaisseur, et la longueur dudit bâtonnet est inférieure à deux fois sa largeur, pour permettre ainsi à un utilisateur de saisir librement ledit bâtonnet entre le pouce et l'index de l'utilisateur à un emplacement adjacent à ladite arête du bâtonnet de sorte que le pouce de l'utilisateur peut être pointé généralement en direction de la paupière.

2. Applicateur selon la revendication 1, comportant une pluralité de stries (24) exposées sur les côtés aplatis dudit bâtonnet.

3. Applicateur selon la revendication 1 ou 2, comportant un liquide absorbé dans ledit tampon.

4. Applicateur selon la revendication 1, 2 ou 3, dans lequel ledit bâtonnet est plus épais sur au moins une portion médiane de ce dernier que le long d'une arête de celui-ci.
